# EUROPEAN PATENT APPLICATION

(11) **EP 4 011 324 A1**
(43) Date of publication of application: **15.06.2022**
(21) Application number: 20213219.7
(22) Date of filing: 10.12.2020
(51) Int. Cl.: A61F 2/06, A61F 2/24, G06F 30/20

(54) **METHOD FOR MANUFACTURING A MOLD FOR A CARDIAC VALVE PROSTHESIS**

(71) Applicant: GrOwnValve GmbH, 10435 Berlin (DE)
(72) Inventor: SCHMITT, Boris, 10435 Berlin (DE)
(74) Representative: Maikowski & Ninnemann Patentanwälte Partnerschaft mbB

(57) **Abstract**

The invention relates to a method for manufacturing a mold for a cardiac valve prosthesis, the method comprising the following steps: a) providing (100) 3-D imaging data of an impaired cardiac valve of an individual, wherein the 3-D imaging data has been previously obtained by an appropriate imaging method; b) 3-D reconstructing (110) the 3-D imaging data, thereby at least partially correcting impairments of the impaired cardiac valve so as to obtain reconstructed 3-D imaging data representing a virtual cardiac valve having a performance that better corresponds to the performance of a non-impaired cardiac valve than the performance of the impaired cardiac valve does, c) using the reconstructed 3-D imaging data to generate (120) a virtual 3-D mold for an individually personalized cardiac valve prosthesis, and d) using the virtual 3-D mold to manufacture (130) a real mold for an individually personalized cardiac valve prosthesis.

## Description

The present invention relates to a method for manufacturing a mold for a cardiac valve prosthesis according to the preamble of claim 1, a computer program product according to the preamble of claim 7 and to a mold for a cardiac valve prosthesis according to the preamble of claim 9.

In prior art, cardiac valve replacement by mechanical and biological cardiac valves is known.

Cardiac valve replacement using mechanical cardiac valves is established since the 1960s. Synthetically manufactured prostheses made from metal or plastic (polymers) are implanted to a patient in an open-heart surgery by use of cardiopulmonary bypass. The disadvantage of this procedure is the necessity of a lifelong anticoagulation therapy to avoid the formation of thrombi on the foreign surface of the prostheses. On the other hand, an advantage is the theoretic lifelong durability of the prosthetic cardiac valve. Therefore, it is mostly used for young adults. Female patients who want to give birth are mostly excluded from this therapy so far.

Biological cardiac valves are mostly produced from bovine or porcine pericardium or consist of whole bovine or porcine cardiac valves. In addition, bovine jugular vein valves are used as cardiac valve prostheses. Valves from animal tissue are called xenogenic. They either are implanted surgically or, since around year 2000, are implanted by cardiac catheter interventions as stented cardiac valves.

Further, biological cardiac valves can be harvested from human valve donors (allografts) or in some cases from the patient herself/himself for use as an autograft (e.g. in the so called ROSS procedure).

The advantage in using biological materials is avoidance of long-term anticoagulation therapy. The disadvantage, however, is that - apart from autografts - these cardiac valves are eventually rejected due to an immune reaction since they originate from a foreign source. This rejection often occurs even though a multistep pretreatment is performed to reduce the risk of an immune reaction. The result of such an immune reaction is degradation of the implanted cardiac valve. Therefore, the durability of biological cardiac valves - especially xenografts - is limited to approximately 5 to 15 years. Afterwards, another cardiac valve replacement is necessary.

The advantage of valves of human origin is a longer durability due to a lower immune response. The main disadvantage is, however, that these valves are increasingly difficult to obtain since the willingness for donations decreases. A special case is the self-donation: By an operation called ROSS procedure, the own (autologous) pulmonary valve is implanted instead of the diseased aortic valve, wherein the missing pulmonary valve is then replaced by a xeno- or allograft referred to above.

As already mentioned, cardiac valve prostheses can be implanted by a surgical or an interventional or a hybrid procedure (combining surgical and interventional techniques). A replacement by an interventional transcutaneous procedure is based on the use of catheters and the modified Seldinger technique. The intervention can be subdivided into a transvenous, transarterial, and a transapical intervention. The transapical access is established through the thoracic wall and the cardiac apex into the left or right ventricle. In case of the transvenous access, the right part of the heart with its valves is reached antegradely (i.e., with the bloodstream). After puncture of the atrial septum, also the left part of the heart can be reached. By the transarterial approach the left heart chambers and valves can be reached retrogradely.

It is an object of the present invention to provide methods and devices that facilitate the manufacture of cardiac valve prostheses that combine a long durability and a low disposition for the formation of thrombi on their surface.

This object is achieved with a method for manufacturing a mold for a cardiac valve prosthesis according to claim 1. This method comprises the steps explained in the following.

First, 3-D imaging data of an impaired cardiac valve of an individual is provided. Thereby, this imaging data has been obtained by an appropriate imaging method prior to carrying out the claimed method. Appropriate methods are, e.g., magnetic resonance imaging (MRI), computed tomography (CT), (3-D) ultrasound (e.g., echocardiography), or 3-D rotational angiography..

Afterwards, the 3-D imaging data is reconstructed in a three-dimensional manner. Thereby, impairments of the impaired cardiac valve are at least partially corrected (so-called virtual valve surgery). As a result, reconstructed 3-D imaging data is obtained that represents a virtual cardiac valve. This virtual cardiac valve has a performance that better corresponds to the performance of a non-impaired cardiac valve than the performance of the impaired cardiac valve does. The term "performance" is in particular to be understood as the ability to open and close in a cardiac blood vessel or heart chamber. Thus, the cardiac valve having a good performance has a good ability to open and close in a cardiac blood vessel or heart chamber.

Subsequently, the reconstructed 3-D imaging data is used to generate a virtual 3-D mold for a cardiac valve prosthesis. Since the reconstructed 3-D imaging data is based on imaging data of a specific individual, the virtual 3-D mold is a mold for an individually personalized cardiac valve prosthesis.

Finally, the virtual 3-D mold is used to manufacture a real mold for a cardiac valve prosthesis. To be more precise, it is used to manufacture a real mold for an individually personalized cardiac valve prosthesis.

This real mold for an individually personalized cardiac valve prosthesis can then be used to manufacture an individually personalized cardiac valve prosthesis. This can be done, e.g., by applying any desired material into the real mold and to give the inserted material the shape of the real mold, e.g., by applying positive or negative pressure, suction or vacuum, temperature changes and/or biochemically active substances such as cross-linkers. To give an example, body tissue of the human or animal in need of obtaining an individually personalized cardiac valve prosthesis can be used for being re-shaped within the real mold as to obtain an individually personalized cardiac valve prosthesis.

In an aspect, the present invention relates to a method that also encompasses a method step for obtaining 3-D imaging data of an impaired cardiac valve of an individual by an appropriate imaging method.

In an embodiment, the performance of the virtual cardiac valve that is represented by the reconstructed 3-D imaging data is tested by simulation after the step of 3-D reconstructing the 3-D imaging data. This simulation is done in terms of systolic and diastolic flow and/or vortices and/or streamlines and/or pressure fields and/or a pressure gradient across the virtual cardiac valve. By such a simulation, the performance of the virtual cardiac valve can be assessed in more detail in order to ensure that the virtual cardiac valve has indeed a better performance than the impaired cardiac valve that is to be replaced. The simulation of the performance of the virtual cardiac valve can best be done by specialized software.

In an embodiment, the 3-D imaging data is the 3-D imaging data of an impaired human cardiac valve.

In an embodiment, the 3-D imaging data is the 3-D imaging data of an impaired animal valve. The animal is, in an embodiment, a rodent or a non-human mammal.

In an embodiment, the step of 3-D reconstruction of the 3-D imaging data comprises a step in which at least one impaired or diseased area of the impaired cardiac valve is virtually excised (so-called virtual valve surgery). Furthermore, the excised area is then replaced by a remodeled area having an appearance of healthy tissue, in particular of healthy cardiac tissue. Then, the reconstructed 3-D imaging data resembles or corresponds to a non-impaired cardiac valve.

While it is generally possible to manufacture the real mold for an individually personalized cardiac valve prosthesis by any appropriate manufacturing method, this step is done, in an embodiment, by injection molding or by 3-D printing. 3-D printing is particular appropriate since it allows for manufacturing individually personalized molds at particularly low cost and high speed.

The real mold for an individually personalized cardiac valve prosthesis can be used to manufacture any kind of cardiac valve prosthesis. Hence, it is, in an embodiment, a mold for an aortic valve prosthesis. In another embodiment, it is a mold for a pulmonary valve prosthesis. In another embodiment, it is a mold for a mitral valve prosthesis. In another embodiment, it is a mold for a tricuspid valve prosthesis.

In an aspect, the present invention also relates to a (non-transitory) computer program product comprising a software with executable code that causes a computer to perform the steps explained in the following upon being executed on the computer. First, provided 3-D imaging data of an impaired cardiac valve of an individual is automatically 3-D reconstructed. Thereby, impairments of the impaired cardiac valve are at least partially corrected. This results in reconstructed 3-D imaging data representing a virtual cardiac valve having a performance that better corresponds to the performance of a non-impaired cardiac valve than the performance of the impaired cardiac valve does.

Furthermore, the computer program causes the computer to automatically use the reconstructed 3-D imaging data to generate a virtual 3-D mold for a cardiac valve prosthesis.

In an embodiment, the software causes the computer additionally to automatically control the manufacturing of a real mold for an individually personalized cardiac prosthesis on the basis of the virtual 3-D mold. Thus, the computer program can control, e.g., a 3-D printer to manufacture a real mold for an individually personalized cardiac valve prosthesis.

In an embodiment, the software causes the computer to perform an additional step prior to automatically generating a virtual 3-D mold for a cardiac valve prosthesis, namely a step of simulating the performance of the virtual cardiac valve in terms of systolic and diastolic flow and/or vortices and/or streamlines and/or pressure fields and/or a pressure gradient across the virtual cardiac valve.

In an aspect, the invention also relates to a mold for an individually personalized cardiac valve prosthesis that is or can be obtained by a method according to the preceding explanations.

All embodiments of the described method can be combined in any desired way and can be transferred either individually or in any arbitrary combination to the described computer program product and the described mold for an individually personalized cardiac valve prosthesis, and vice versa.

Further details of aspects of the present invention will be explained in the following with respect to exemplary embodiments and accompanying Figures. In the Figures:
- Figure 1: shows a flowchart of a method for manufacturing a mold for a cardiac valve prosthesis;
- Figure 2: shows an exemplary embodiment of such a mold;
- Figure 3: shows the mold of Figure 2 with further components;
- Figure 4: shows a first step of an application method of the mold of Figure 2;
- Figure 5: shows a second step of an application method of the mold of Figure 2;
- Figure 6: shows a third step of an application method of the mold of Figure 2;
- Figure 7: shows a fourth step of an application method of the mold of Figure 2; and
- Figure 8: shows a fifth step of an application method of the mold of Figure 2.

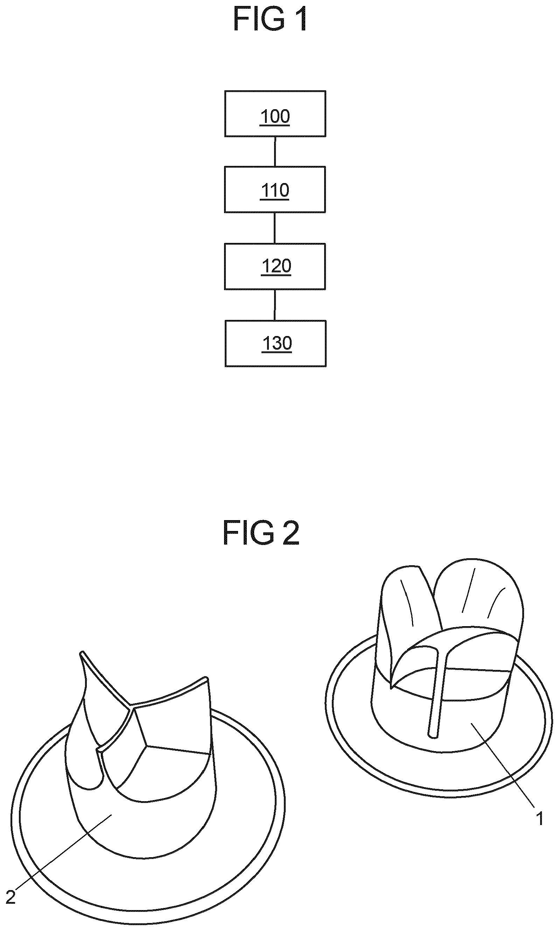

Figure 1 shows a flowchart of an exemplary embodiment of a method for manufacturing a mold for a cardiac valve prosthesis. In a first step 100, 3-D imaging data of an impaired human cardiac valve is provided.

In a second step 110, the 3-D imaging data is reconstructed. This second step 110 can also be denoted as virtual cardiac valve surgery. By this step, a detected impairment of the human cardiac valve from which the 3-D imaging data was obtained is virtually corrected. This impairment typically results in a more or less pronounced dysfunction of the human cardiac valve. When performing the virtual cardiac valve surgery 110, the according impairment is virtually correct. Thus, reconstructed 3-D imaging data results that represents a human cardiac valve having better functional properties than the human cardiac valve from which the 3-D imaging data was obtained. Expressed in other words, the cardiac valve of the reconstructed 3-D imaging data has a better functionality than the original cardiac valve from which the 3-D imaging data has been obtained.

In a third step 120, the reconstructed 3-D imaging data is used for generating a virtual 3-D mold for a personalized cardiac valve prosthesis.

Subsequently, the virtual 3-D mold is used in a fourth step 130 to manufacture a real mold for a cardiac valve prosthesis. This real mold will then serve for manufacturing a cardiac valve prosthesis having better properties than the cardiac valve from which the 3-D imaging data has been obtained.

Figure 2 shows an exemplary embodiment of a mold for manufacturing a cardiac valve prosthesis. This mold comprises a first part 1 and a second part 2 that can be fit into each other. The first part 1 and the second part 2 act together as negative and positive form of a cardiac valve prosthesis to be manufactured.

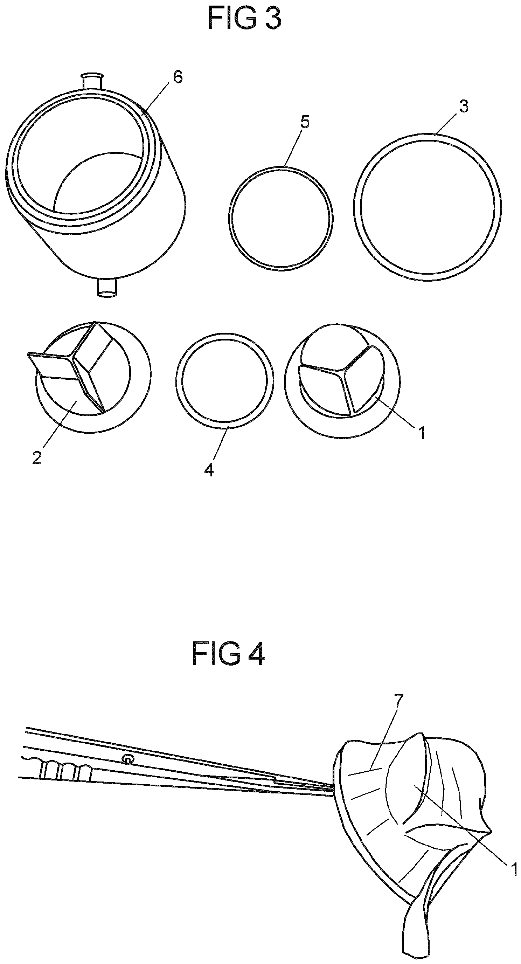

Figure 3 shows the first part 1 and the second part 2 of the mold depicted in Figure 2 with further components, namely a plate 3, a ring 4, a gasket 5 and a reaction container 6. These components are typically provided in a set. It is possible to assemble the first part 1 and the second part 2 of the mold after having placed cardiac tissue between them. This cardiac tissue is then hold in place by the ring 4. Afterwards, the assembly of the first part 1, the second part 2, the cardiac tissue and a gasket 5 can be placed into the reaction container 6. The plate 3 serves for assisting in manipulation steps during proper placement of cardiac tissue between the first part 1 and the second part 2 of the mold.

The individual steps of manufacturing a cardiac valve prosthesis with the help of the first part 1 and the second part 2 of the mold will be explained in the following in more detail making reference to Figures 4 to 8. The same numeral references will be used in all Figures for explaining the same or similar elements.

As shown in Figure 4, pericardium 7 or other cardiac tissue is tightly drawn above the first part 1 of the mold. This is done with the help of a pair of tweezers 8. However, other manipulation instruments can also be used.

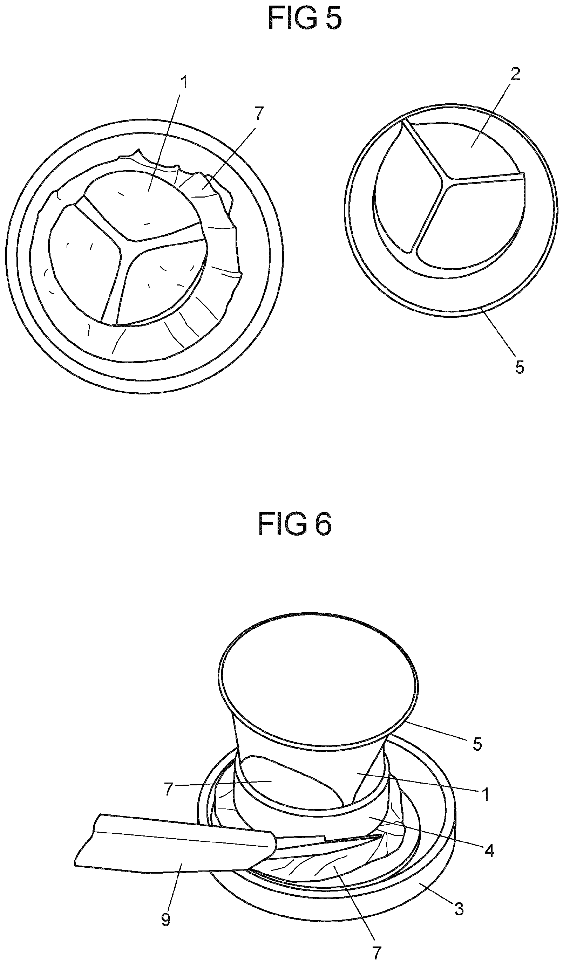

As shown in a top view in Figure 5, the pericardium 7 then fully covers the first part 1 of the mold. In contrast, the second part 2 of the mold is not yet covered with pericardium. However, the gasket 5 is already placed around the first part 1 of the mold.

As shown in Figure 6, the second part 2 is then placed above the first part 1 of the mold, wherein the pericardium 7 is pressed between the first part 1 and the second part 2 of the mold. Furthermore, the ring 4 serves for keeping the pericardium 7 in place. Pericardium 7 protruding from the second part 2 of the mold and from the ring 4 is cut with a scalpel 9. This is done on the plate 3 providing a clean and plane cutting surface.

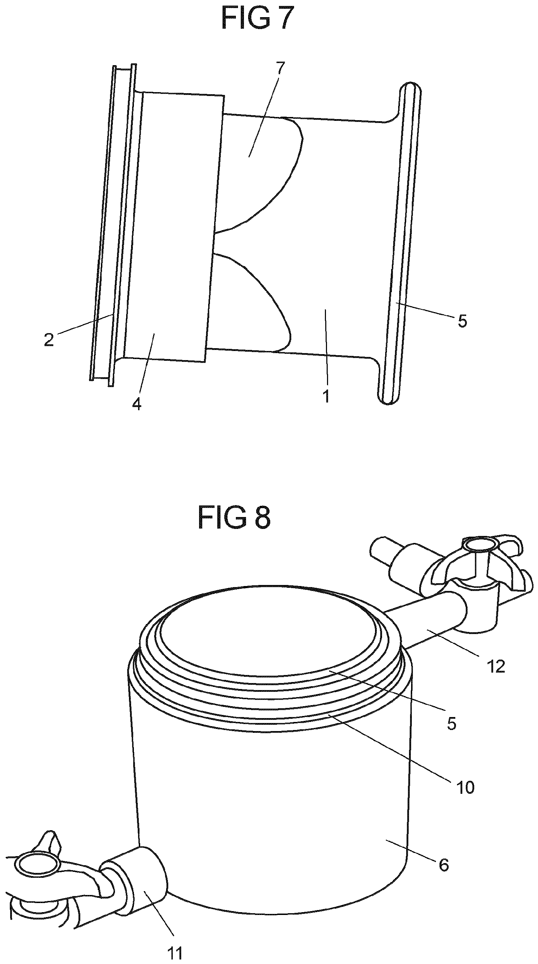

Figure 7 shows the fully assembled mold with the first part 1 of the mold overlaying the second part 2 of the mold and the ring 4 securing the pericardium 7 on the mold. This fully assembled mold can then be placed into the reaction container 6, as shown in Figure 8. A lid (e.g., the plate 3 shown in Figure 3) can be placed on top of the reaction container 6. The gasket 5 and a reaction container gasket 10 will then serve for a sufficient sealing between an interior of the reaction container 6 and an exterior.

The reaction container 6 comprises an inlet port 11 and an outlet port 12. A reaction liquid can be fed into the reaction container 6 through the inlet port 11 and can be drained from an interior of the reaction container 6 by the outlet 12.

In the embodiment shown in Figure 8, a reaction liquid comprising a cross-linker is fed into the interior of the reaction container 6. This cross-linker serves for cross-linking the pericardium 7 so as to keep it in the form given to the pericardium 7 by the mold. Thus, when releasing the first part 1 of the mold from the second part 2 of the mold, the pericardium 7 still retains the shape that has been given to it by the first part 1 of the mold and the second part 2 of the mold, i.e. the shape of a cardiac valve. After separation of the leaflets, the shaped pericardium 7 can subsequently be used as fully functional cardiac valve prosthesis.

## Claims

1. Method for manufacturing a mold for a cardiac valve prosthesis, the method comprising the following steps:
a) providing (100) 3-D imaging data of an impaired cardiac valve of an individual, wherein the 3-D imaging data has been previously obtained by an appropriate imaging method,
b) 3-D reconstructing (110) the 3-D imaging data, thereby at least partially correcting impairments of the impaired cardiac valve so as to obtain reconstructed 3-D imaging data representing a virtual cardiac valve having a performance that better corresponds to the performance of a non-impaired cardiac valve than the performance of the impaired cardiac valve does,
c) using the reconstructed 3-D imaging data to generate (120) a virtual 3-D mold for an individually personalized cardiac valve prosthesis, and
d) using the virtual 3-D mold to manufacture (130) a real mold (1, 2) for a cardiac valve prosthesis.

2. Method according to claim 1, **characterized in that** after step b) a step of simulating the performance of the virtual cardiac valve in terms of at least one of systolic and diastolic flow, vortices, streamlines, pressure fields and pressure gradients across the virtual cardiac valve is performed.

3. Method according to claim 1 or 2, **characterized in that** the 3-D imaging data is the 3-D imaging data of an impaired cardiac valve of a human.

4. Method according to claim 1 or 2, **characterized in that** the 3-D imaging data is the 3-D imaging data of an impaired cardiac valve of an animal, in particular of a rodent or a non-human mammal.

5. Method according to any of the preceding claims, **characterized in that** 3-D reconstructing (110) the 3-D imaging data comprises virtually excising at least one impaired or diseased area of the impaired cardiac valve and remodeling the excised area to have an appearance of healthy tissue.

6. Method according to any of the preceding claims, **characterized in that** the real mold (1, 2) for an individually personalized cardiac valve prosthesis is manufactured by injection molding or by 3-D printing.

7. Method according to any of the preceding claims, **characterized in that** the real mold (1, 2) for an individually personalized cardiac valve prosthesis is a mold for an aortic valve prosthesis, for a pulmonary valve prosthesis, for a mitral valve prosthesis, or for a tricuspid valve prosthesis.

8. Computer program product comprising a software with executable code that, when executed on a computer, causes the computer to perform the following steps:
a) automatically 3-D reconstructing (110) provided 3-D imaging data of an impaired cardiac valve of an individual, thereby at least partially correcting impairments of the impaired cardiac valve so as to obtain reconstructed 3-D imaging data representing a virtual cardiac valve having a performance that better corresponds to the performance of a non-impaired cardiac valve than the performance of the impaired cardiac valve does, and
b) automatically using the reconstructed 3-D imaging data to generate (120) a virtual 3-D mold for a cardiac valve prosthesis.

9. Computer program product according to claim 8, **characterized in that** the software causes the computer to perform the following additional step:
c) automatically controlling manufacturing (130) a real mold (1, 2) for a cardiac valve prosthesis on the basis of the virtual 3-D mold.

10. Computer program product according to claim 8 or 9, **characterized in that** the software causes the computer to perform the following additional step prior to carrying out step b) of claim 8: simulating the performance of the virtual cardiac valve in terms of at least one of systolic and diastolic flow, vortices, streamlines, pressure fields and pressure gradients across the virtual cardiac valve.

11. Mold for an individually personalized cardiac valve prosthesis obtainable by a method according to any of claims 1 to 7.
